(19) Europäisches Patentamt
European Patent Office
Office européen des brevets

(11) Publication number: 0 372 935 A2

# (12) EUROPEAN PATENT APPLICATION

(21) Application number: 89312727.4

(51) Int. Cl.5: C07D 209/48

(22) Date of filing: 06.12.89

(30) Priority: 06.12.88 JP 306823/88

(43) Date of publication of application: 13.06.90 Bulletin 90/24

(84) Designated Contracting States: BE DE FR GB IT NL

(71) Applicant: MITSUBISHI GAS CHEMICAL COMPANY, INC.
5-2, Marunouchi 2-chome Chiyoda-Ku
Tokyo, 100(JP)

(72) Inventor: Tanisake, Hiroka Polymer Res. Inst.
Mitsubishi Gas
Chemical Co. Inc. 6-2, Higashiyahata
5-chome
Hiratsuka-shi Kanagawa-ken(JP)
Inventor: Toki, Tsukasa Polymer Res. Inst.
Mitsubishi Gas
Chemical Co. Inc. 6-2, Higashiyahata
5-chome
Hiratsuka-shi Kanagawa-ken(JP)

(74) Representative: Myerscough, Philip Boyd et al
J.A.Kemp & Co. 14, South Square Gray's Inn
London, WC1R 5EU(GB)

## (54) Diimidodicarboxylic acids and process for the productionn thereof.

(57) New diimidodicarboxylic acids are represented by the following formula

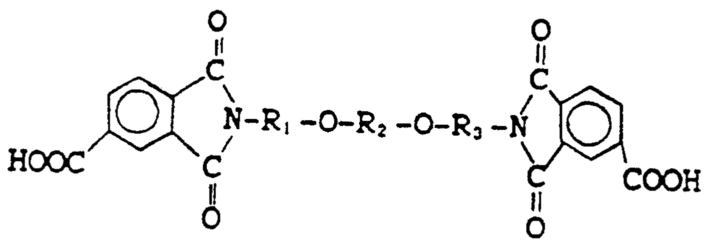

wherein $R_1$ and $R_3$ each represent an aromatic hydrocarbon having 6 carbon atoms, and $R_2$ represents an aromatic hydrocarbon having 6 carbon atoms, or an aromatic hydrocarbon having 12 carbon atoms which is optionally interrupted by a sulfonyl group, isopropylidene group or hexafluoroisopropylidene group. These compounds may be produced by a process which comprises reacting a trimellitic anhydride with an aromatic diamine represented by the following formula

$H_2N\text{-}R_1\text{-}O\text{-}R_2\text{-}O\text{-}R_3\text{-}NH_2$

wherein $R_1$ and $R_3$ each represent an aromatic hydrocarbon having 6 carbon atoms, and $R_2$ represents an aromatic hydrocarbon having 6 carbon atoms, or an aromatic hydrocarbon having 12 carbon atoms which is optionally interrupted by a sulfonyl group, isopropylidene group or hexafluoroisopropylidene group.
The compounds may be used to prepare polymers which have a high heat resistance.

EP 0 372 935 A2

## Diimidodicarboxylic acids and process for the production thereof

### FIELD OF THE INVENTION

This invention relates to diimidodicarboxilic acids which are formed of trimellitic anhydride and primary diamines containing at least two oxygen bonds and have excellent heat resistance, and a process for the production thereof.

Diimidodicarboxylic acids are considered industrially important as a dicarboxylic acid component for polymers having excellent heat resistance such as polyamide-imide resins, polyester imides, etc. The diimidodicrboxylic acids of the present invention give polymers having further better heat resistance.

### PRIOR ART OF THE INVENTION

Diimidodicarboxylic acids are synthesized from polycarboxylic anhydride or a ring-opened adduct thereof and diamines containing primary amino groups, and in general, have the following structural formula

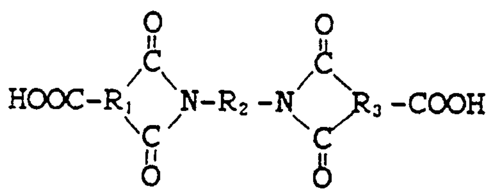

wherein $R_1$, $R_2$ and $R_3$ each represent an aromatic or aliphatic cyclic hydrocarbon.

Japanese Patent Publication No. 21500/1963, U.S. Patent 3697471 and U.S. Patent 3929714 are examples using these diimidodicarboxylic acids as a dicarboxylic acid component of polymers. Further, U.S. Patent 4145351 and German Patent 1795596 disclose diimidodicarboxylic acids. Japanese Patent Publication No. 21956/1986 also dislcoses diimidopolycarboxylic acids as a component of allyl esters.

In Japanese Patent Publication No. 21500/1963, U.S. Patent 3697471, U.S. Patent 3929714 and German Patent 1795596, diimidodicarboxylic acids are formed of trimellitic anhydrides as a polycarboxylic acid component and diaminodiphenylmethane, diaminodiphenyl ether, diaminodiphenyl sulfone or p-phenylenedimaine as a primary diamine.

In Japanese Patent Publication No. 21956/1986, a wide variety of polycarboxylic anhydrides are used as a component of diimidopolycarboxylic acids. However, examples of the diamine component are limited to the above.

### SUMMARY OF THE INVENTION

It is an object of the present invention to provide a novel diimidodicarboyxlic acid and a process for the production thereof.

It is another object of the present invention to provide a novel diimidodicarboxylic acid having high heat resistance and a process for the production thereof.

It is further another object of the present invention to provide a novel diimidodicarboxylic acid suitable as a material for polymers having excellent heat resistance, and a process for the production thereof.

It is yet another object of the present invention to provide a novel diimidodicarboxylic acid which can give polymers having further better heat resistance than polymers obtained from conventionally known diimidodicarboxylic acids.

According to the present invention, there is provided a diimidodicarboxylic acid represented by the following formula

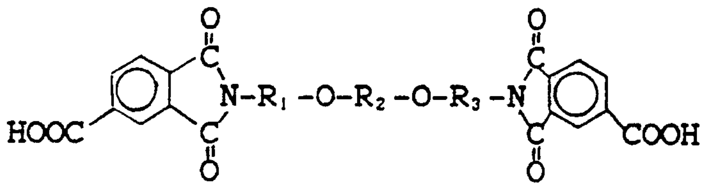

wherein $R_1$ and $R_3$ each represent an aromatic hydrocarbon having 6 carbon atoms, and $R_2$ represents an aromatic hydrocarbon having 6 or 12 carbon atoms, provided that when the aromatic hydrocarbon has 12 carbon atoms, it may be interrupted by a sulfonyl group, isopropylidene group or hexafluoroisopropylidene group. The aromatic hydrocarbon group having 6 carbon atoms is typically phenylene. The aromatic hydrocarbon group having 12 carbon atoms typically comprises two aromatic rings, optionally separated by intervening groups. Preferably the aromatic hydrocarbon group having 12 carbon atoms comprises two phenylene rings, optionally separated by intervening groups.

According to the present invention, there is also provided a process for the production of the above diimidodicarboxylic acid, which comprises reacting an aromatic diamine represented by the following formula

$H_2N\text{-}R_1\text{-}O\text{-}R_2\text{-}O\text{-}R_3\text{-}NH_2$

wherein $R_1$ and $R_3$ each represent an aromatic hydrocarbon having 6 carbon atoms, and $R_2$ represents an aromatic hydrocarbon having 6 to 12 carbon atoms, provided that when the aromatic hydrocarbon has 12 carbon atoms, it may be interrupted by a sulfonyl group, isopropylidene group or hexafluoroisopropylidene group, with a trimellitic anhydride.

Figure 1 to Figure 6 show infrared absorption spectra of diimidodicarboxylic acids obtained in Examples 1 to 6.

Figures 7 to 12 are charts for data of thermogravimetric analysis of diimidodicarboxylic acids obtained in Examples 1 to 6.

Figures 13 to 14 are charts for data of thermogravimetric analysis of diimidodicarboxylic acids obtained in Comparative Examples 1 to 2.

The present inventors have made a diligent study to develop novel diimidodicarboxylic acids as a material for heat-resistant polymers, and found that novel diimidodicarboxylic acids suitable as a material for heat-resistant polymers can be produced at high yield and high purity by a reaction between a primary diamine having at least two oxygen bonds in the molecule and a trimellitic anhydride.

Embodiments of the diimidodicarboxylic acids of the present invention are as described below.

(A) A diimidodicarboxylic acid represented by the formula

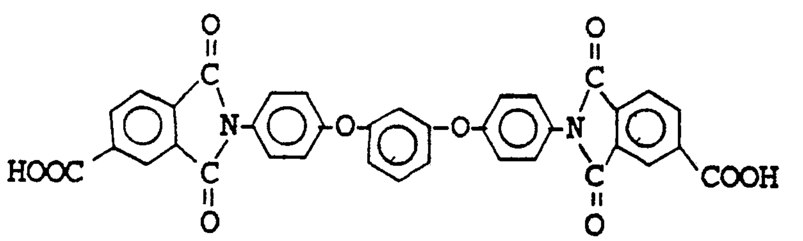

(B) A diimidodicarboxylic acid represented by the formula

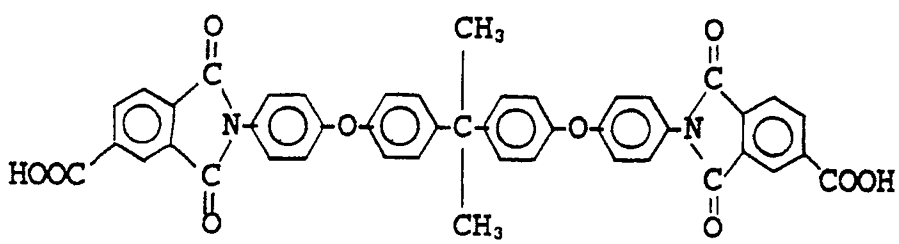

(C) A diimidodicarboxylic acid represented by the formula

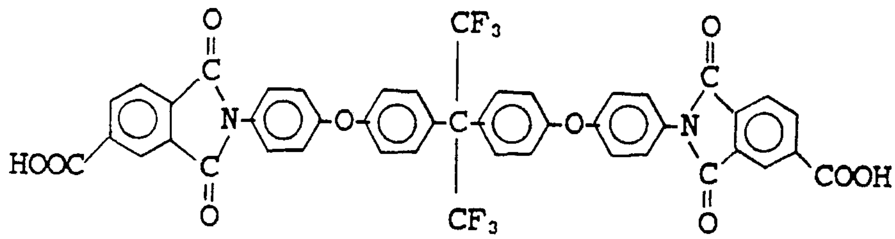

(D) A diimidodicarboxylic acid represented by the formula

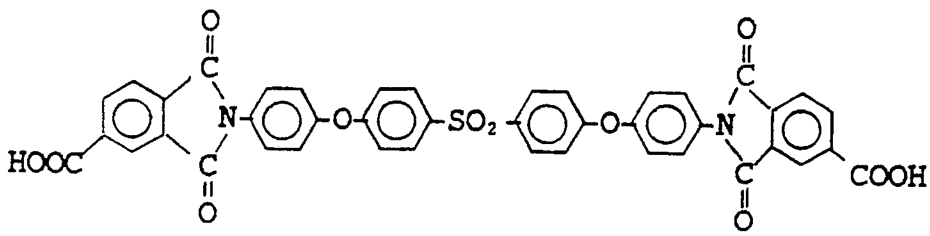

(E) A diimidodicarboxylic acid represented by the formula

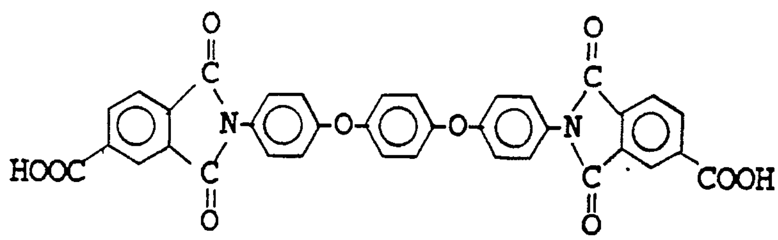

(F) A diimidodicarboxylic acid represented by the formula

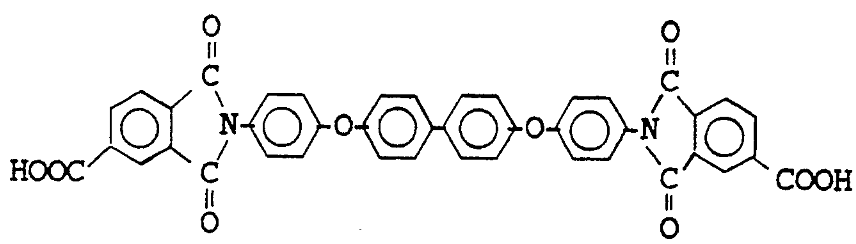

Examples of the primary diamine having at least two ether groups in the molecule, usable in the present invention, are as follows. However, the primary diamine shall not be limited thereto.

1,3-bis(4-aminophenoxy)benzene, 1,4-bis(4-aminophenoxy)benzene, 2,2-bis{4-(4-aminophenoxy)-phenyl}-propane, 2,2-bis{4-(4-aminophenoxy)phenyl}hexafluoropropane, 4,4′-bis(4-aminophenoxy)diphenyl sulfone, 4,4′-bis(4-aminophenoxy)diphenyl, etc.

In the present invention, diimidodicarboxylic acids are synthesized in an ordinary organic solvent, and preferably in a solvent inert to carboxyl group, acid anhydride group and amino group. For example, ordinary solvents such as cresol, dimethylacetamide, dimethylformamide, dimethylsulfoxide, N-methylpyrrolidone, etc., are usable. The molar ratio of the primary diamine to the trimellitic anhydride is preferably 1:2.

The temperature for the reaction between the primary diamine and the trimellitic anhydride is not specially limited, and the reaction can be carried out at a temperature between 20 and 300° C. The solvent is used, in general, in such an amount that it contains 90 to 10 % by weight of a reaction product, i.e. diimidodicarboxylic acid.

The reaction is carried out until no water is formed as a by-product, and the reaction time is usually

between 4 and 7 hours in the above temperature range and suitably adjustable depending upon the reaction temperatures.

In the reaction for formation of diimidodicarboxylic acid, trimellitic anhydride and primary diamine react with each other to form an amic acid first. When the amic acid is ring-closed into an imide, water is formed as a by-product. The existence of the formed water in the reaction system is undesirable since it causes hydrolysis of imide groups. The formed water is usually removed from the system through a partial condenser, etc., and can be also removed smoothly by azeotropic distillation using a solvent such as xylene, toluene, etc.

Formed diimidodicarboxylic acids are insolube, or only have a very low solubility, in most of organic solvents, and a reaction liquid is in a slurry state after a theoretical amount of the formed water is removed. Concerning the above-specified solvents, diimidodicarboxylic acid is hardly dissolved in cresol, and is slightly dissolved in aprotic organic solvents such as dimethylacetamide, dimethylformamide, N-methylpyrrolidone, dimethyl sulfoxide, etc.

In case of using a solvent in which diimidodicarboxylic acid is insoluble such as cresol, usual post-reaction procedures such as filtering, washing, drying, etc., are sufficient to obtain diimidodicarboxylic acid. On the other hand, when an aprotic organic solvent is used, diimidodicarboxylic acid is obtained by pouring a reaction liquid into a solvent in which the diimidodicarboxylic acid is insoluble, such as lower alcohol, water, etc., to precipitate the diimidodicarboxylic acid and then carrying out usual procedures such as filtering, washing, drying, etc. In the above procedures, diimidodicarboxylic acid can be obtained at very high yields. And diimidodicarboxylic acid obtained in the above procedure has very high purity even without adding any special purification procedure.

The diimidodicarboxylic acid of the present invention has an advantage that its heat resistance is higher than that of conventional diimidodicarboxylic acids. Further, the diimidodicarboxylic acid of the present invention achieves the effect that polymers using the diimidodicarboxylic acid of the present invention have high heat resistance and are easily moldable.

## EXAMPLES

The present invention will be specifically explained by reference to Examples hereinbelow, although it shall not be limited thereto.

In the following Examples, the method for thermogravimetric analysis is as follows:

The analysis was effected by using TG/DTA 200 manufactured by Seiko Electronics K.K., and 2 to 20 mg of a sample at a dry air supply amount of 300 ml/min., and a temperature elevation ratio of 10 °C/min.

TG(°C) stands for a weight decrease ratio of a sample at each temperature.

DTG(%/min.) stands for a differentiation value of a weight decrease per time during temperature elevation.

$T_5$% loss (°C) stands for a temperature when the weight of a sample is decreased by 5 %.

The method for measurement of endothermic temperature (Tm, °C) is as follows:

A differential scanning calorimeter (DSC), model SSC/560S, manufactured by Seiko Electronics K.K., was used, and the measurement was carried out by placing 10 mg of a sample in an unsealed container of aluminum under a nitrogen gas current of 30 ml/min., and at a temperature elevation ratio of 20 °C/min.

## EXAMPLE 1

A one-liter four-necked separable flask having a partial condernser, a thermometer and a nitrogen-introducing tube was charged with 96 g (0.5 mole) of trimellitic acid and 417 g of cresol, and the trimellitic acid was dissolved in the cresol with stirring at 100 °C. Then, 73 g (0.25 mole) of 1,3-bis(p-aminophenoxy)-benzene was added to show a temperature increase of about 10 °C under an exothermic reaction. And at the same time, the temperature of the mixture was elevated to 150 °C to start a reaction. Several minutes after the temperature reached 150 °C, precipitation of yellow crystals was observed, and water formed together with imidation started to be distilled off. The formed water was removed out of the system through the partial condenser. The reaction was continued, and when no water was distilled off, the reaction was stopped. The time required for the reaction was 6 hours long.

The reaction mixture after the reaction was in a slurry state due to precipitation of yellow crystals. The reaction mixture was filtered, and the resultant solid was fully washed with ethanol, and then dried under reduced pressure to give 155.95 g of yellow crystals of 4,4'-(1,3-diphenoxybenzene)-bis(N-trimellitimide).

The yield thereof based on 1,3-bis(p-aminophenoxy)benzene was 97.5 mole%.

The crystals had a melting point of 356 °C, and showed infrared absorption spectra of carboxyl group $\nu_{O-H}$ at 2,500~3,000 $cm^{-1}$ and carboxyl group $\nu_{C=O}$ at 1,695 $cm^{-1}$ and abosorption spectrum of imide group at 1,780, 1,720 and 730 $cm^{-1}$. Figure 1 shows infrared absorption spectra of these crystals.

The crystals had an acid number of 174.5 mgKOH/g, which was well in agreement with a theoretical value of 175 mgKOH/g. Elemental analysis of this product showed C-67.20 %, H-3.35 % and N-4.53 %, which were well in agreement with theoretical values (C-67.5 %, H-3.15 % and N-4.37 %), and it was therefore shown that the product was 4,4′-(1,3-diphenoxybenzene)-bis(N-trimellitimide).

Further, the temperature at which the product started thermal decomposition was 402.3 °C. Figure 7 shows a chart of thermogravimetric analysis of these crystals.

EXAMPLE 2

A one-litter four-necked separable flask having a partial condenser, a thermometer and a nitrogen-introducing tube was charged with 96 g (0.5 mole) of trimellitic anhydride and 417 g of cresol, and the trimellitic anhydride was dissolved in the cresol with stirring at 100 °C. Then, 73 g (0.25 mole) of 1,4-bis(p-aminophenoxy)benzene was added to show a temperature increase of 10 °C under an exothermic reaction. And at the same, the temperature of the mixture was elevated to 150 °C to start a reaction. Several minutes after the temperature reached 150 °C, precipitation of yellow crystals was observed, and water formed together with imidation started to be distilled off. The formed water was removed out of the system through the partial condenser. The reaction was continued, and when no water was distilled off, the reaction was stopped. The time required for the reaction was 6 hours long.

The reaction mixture after the reaction was in a slurry state due to precipitation of yellow crystals. The reaction mixture was filtered, and the resultant solid was fully washed with ethanol, and then dried under reduced pressure to give 155.95 g of yellow crystals of 4,4′-(1,4-diphenoxybenzene)-bis(N-trimellitimide). The yield thereof based on 1,4-bis(p-aminophenoxy)benzene was 97.5 mole%.

The crystals had a melting point of 408 °C, and showed infrared absorption spectra of carboxyl group $\nu_{O-H}$ at 2,500~3,000 $cm^{-1}$ and carboxyl group $\nu_{C=O}$ at 1,690 $cm^{-1}$ and absorption spectrum of imide group at 1,780, 1,720 and 730 $cm^{-1}$. Figure 2 shows infrared absorption spectra of these crystals.

The crystals had an acid number of 176.5 mgKOH/g, which was well in agreement with a theoretical value of 175 mgKOH/g. Elemental analysis of this product showed C-67.30 %, H-3.25 % and N-4.50 %, which were well in agreement with theoretical values (C-67.5 %, H-3.15 % and N-4.37 %), and it was therefore shown that the product was 4,4′-(1,4-diphenoxybenzene)-bis(N-trimellitimide).

Further, the temperature at which the product started thermal decomposition was 410.6 °C. Figure 8 shows a chart of thermogravimetric analysis of the product.

EXAMPLE 3

A one-litter four-necked separable flask having a partial condenser, a thermometer and a nitrogen-introducing tube was charged with 76.8 g (0.4 mole) of trimellitic anhydride and 500 g of cresol, and the trimellitic anhydride was dissolved in the cresol with stirring at 100 °C. Then, 82 g (0.2 mole) of 2,2-bis(4-aminophenoxyphenyl)-propane was added to show a temperature increase of 10 °C under an exothermic reaction. And at the same, the temperature of the mixture was elevated to 155 °C to start a reaction. Several minutes after the temperature reached 155 °C, precipitation of yellow crystals was observed, and water formed together with imidation started to be distilled off. The formed water was removed out of the system through the partial condenser. The reaction was continued, and when no water was distilled off, the reaction was stopped. The time required for the reaction was 5 hours long.

The reaction mixture after the reaction was in a slurry state due to precipitation of yellow crystals. The reaction mixture was filtered, and the resultant solid was fully washed with ethanol, and then dried under reduced pressure to give 144.15 g of yellow crystals of {bis(4-diphenylether)2,2-propane}-bis(N-trimellitimide). The yield thereof based on 2,2-bis(4-aminophenoxyphenyl)propane was 95.8 mole%.

The crystals had a melting point of 335 °C, and showed infrared absorption spectra of carboxyl group $\nu_{O-H}$ at 2,500~3,000 $cm^{-1}$ and carboxyl group $\nu_{C=O}$ at 1,710 $cm^{-1}$ and abosorption spectrum of imide group at 1,780, 1,720 and 730 $cm^{-1}$. Figure 3 shows infrared absorption spectra of these crystals.

The crystals had an acid number of 149.0 mgKOH/g, which was well in agreement with a theoretical value of 148 mgKOH/g. Elemental analysis of this product showed C-70.62 %, H-4.02 % and N-3.59 %,

which were well in agreement with theoretical values (C-71.23 %, H-4.00 % and N-3.69 %), and it was therefore shown that the product was {bis(4-diphenylether)-2,2-propane}-bis(N-trimellitimide).

Further, the temperature at which the product started thermal decomposition was 407.4° C. Figure 9 shows a chart of thermogravimetric analysis of the product.

EXAMPLE 4

A one-litter four-necked separable flask having a partial condenser, a thermometer and a nitrogen-introducing tube was charged with 76.8 g (0.4 mole) of trimellitic anhydride and 500 g of cresol, and the trimellitic anhydride was dissolved in the cresol with stirring at 100° C. Then, 86.5 g (0.2 mole) of 4,4′-bis(4-aminophenoxy)-diphenyl sulfone was added to show a temperature increase of 10° C under an exothermic reaction. And at the same, the temperature of the mixture was elevated to 160° C to start a reaction. About one hour after the temperature reached 160° C, precipitation of yellow crystals was observed, and water formed together with imidation started to be distilled off. The formed water was removed out of the system through the partial condenser. The reaction was continued, and when no water was distilled off, the reaction was stopped. The time required for the reaction was 5 hours long.

The reaction mixture after the reaction was in a slurry state due to precipitation of yellow crystals. The reaction mixture was filtered, and the resultant solid was fully washed with ethanol, and then dried under reduced pressure to give 145.1 g of light yellow crystals of {4,4′-bis(4,4′-diphenoxy)diphenyl sulfone}-bis-(N-trimellitimide). The yield thereof based on 4,4′-bis(4-aminophenoxy)diphenyl sulfone was 93.0 mole%. The crystals had a melting point of 348° C, and showed infrared absorption spectra of carboxyl group $\nu_{O-H}$ at 2,500~3,000 $cm^{-1}$ and carboxyl group $\nu_{C=O}$ at 1,680 $cm^{-1}$ and abosorption spectrum of imide group at 1,780, 1,720 and 730 $cm^{-1}$. Figure 4 shows infrared absorption spectra of these crystals.

The crystals had an acid number of 143.3 mgKOH/g, which was well in agreement with a theoretical value of 143.9 mgKOH/g. Elemental analysis of this product showed C-64.7 %, H-3.11 % and N-3.57 %, which were well in agreement with theoretical values (C-64.61 %, H-3.10 % and N-3.59 %), and it was therefore shown that the product was {4,4′-bis(4,4′-diphenoxy)diphenyl sulfone}-bis(N-trimellitimide).

Further, the temperature at which the product started thermal decomposition was 403.0° C. Figure 10 shows a chart of thermogravimetric analysis of the product.

EXAMPLE 5

A one-litter four-necked separable flask having a partial condenser, a thermometer and a nitrogen-introducing tube was charged with 76.8 g (0.4 mole) of trimellitic anhydride and 500 g of cresol, and the trimellitic anhydride was dissolved in the cresol with stirring at 100° C. Then, 73.7 g (0.2 mole) of 4,4′-bis(4-aminophenoxy)-diphenyl was added to show a temperature increase of 10° C under an exothermic reaction. And at the same, the temperature of the mixture was elevated to 160° C to start a reaction. Immediately after the addition of the diamine, precipitation of yellow crystals was observed, and water formed together with imidation started to be distilled off. The formed water was removed out of the system through the partial condenser. The reaction was continued, and when no water was distilled off, the reaction was stopped. The time required for the reaction was 5 hours long.

The reaction mixture after the reaction was in a slurry state due to the precipitation of yellow crystals. The reaction mixture was filtered, and the resultant solid was fully washed with ethanol, and then dried under reduced pressure to give 139.5 g of yellow crystals of {4,4′-bis(4,4′-diphenoxy)diphenyl}-bis(N-trimellitimide). The yield thereof based on 4,4′-bis(4-aminophenoxy)diphenyl was 97.3 mole%.

The crystals had a melting point of 416° C, and showed infrared absorption spectra of carboxyl group $\nu_{O-H}$ at 2,500~3,000 $cm^{-1}$ and carboxyl group $\nu_{C=O}$ at 1,710 $cm^{-1}$ and abosorption spectrum of imide group at 1,780, 1,720 and 730 $cm^{-1}$. Figure 5 shows infrared absorption spectra of these crystals.

The crystals had an acid number of 157.0 mgKOH/g, which was well in agreement with a theoretical value of 156.7 mgKOH/g. Elemental analysis of this product showed C-70.70 %, H-3.34 % and N-3.86 %, which were well in agreement with theoretical values (C-70.39 %, H-3.38 % and N-3.91 %), and it was therefore shown that the product was {4,4′-bis(4,4′-diphenoxy)diphenyl}-bis(N-trimellitimide).

Further, the temperature at which the product started thermal decomposition was 408.1° C. Figure 11 shows a chart of thermogravimetric analysis of the product.

EXAMPLE 6

A one-litter four-necked separable flask having a partial condenser, a thermometer and a nitrogen-introducing tube was charged with 71.4 g (0.372 mole) of trimellitic anhydride and 500 g of cresol, and they were dissolved with stirring at 100° C. Then, 96.5 g (0.186 mole) of 2,2-bis(4-aminophenoxy-phenyl)-hexafluoropropane was added to show a temperature increase of 10° C under an exothermic reaction. And at the same, the temperature of the mixture was elevated to 160° C to start a reaction. Several minutes after the temperature reached 160° C, precipitation of yellow crystals was observed, and water formed together with imidation started to be distilled off. The formed water was removed out of the system through the partial condenser. The reaction was continued, and when no water was distilled off, the reaction was stopped. The time required for the reaction was 5 hours long.

The reaction mixture after the reaction was in a slurry state due to the precipitation of yellow crystals. The reaction mixture was filtered, and the resultant solid was fully washed with ethanol, and then dried under reduced pressure to give 154.3 g of yellow crystals of {bis(4-diphenyl ether)-2,2-hexafluoropropane}-bis(N-trimellitimide). The yield thereof based on 2,2-bis(4 aminophenoxyphenyl)-hexafluoropropane was 95.8 mole%.

The crystals had a melting point of 341° C, and showed infrared absorption spectra of carboxyl group $\nu_{O\text{-}H}$ at 2,500~3,000 $cm^{-1}$ and carboxyl group $\nu_{C=O}$ at 1,680 $cm^{-1}$ and abosorption spectrum of imide group at 1,780, 1,720 and 720 $cm^{-1}$. Figure 6 shows infrared absorption spectra of these crystals.

The crystals had an acid number of 130.9 mgKOH/g, which was well in agreement with a theoretical value of 129.6 mgKOH/g. Elemental analysis of this product showed C-62.60 %, H-2.81 % and N-3.20 %, which were well in agreement with theoretical values (C-62.36 %, H-2.79 % and N-3.23 %), and it was therefore shown that the product was {bis(4-dipheny ether)2,2-hexafluoropropane}-bis(N-trimellitimide).

Further, the temperature at which the product started thermal decomposition was 402.3° C. Figure 12 shows a chart of thermogravimetric analysis of the product.

COMPARATIVE EXAMPLE 1

4,4′-Diphenylmethane-bis(N-trimellitimide) was synthesized by repeating Example 1 except that 4,4′-diaminodiphenylmethane was used in place of the diamine in Example 1. The yield of the 4,4′-diphenylmethane-bis(N-trimellitimide) was 96.8 mole%. The temperature at which this product started thermal decomposition was 378.9° C. Figure 13 shows a chart of thermogravimetric analysis of the product.

COMPARATIVE EXAMPLE 2

4,4′-Diphenyl ether-bis(N-trimellitimide) was synthesized by repeating Example 1 except that 4,4′-diaminodiphenyl ether was used in place of the diamine in Example 1. The yield of the 4,4′-diphenyl ether-bis(N-trimellitimide) was 96.6 mole%. The temperature at which this product started thermal decomposition was 386.6° C. Figure 14 shows a chart of thermogravimetric analysis of the product.

REFERENTIAL EXAMPLE A

A 50 milliliter reactor equipped with a stirrer, thermometer, pressure gage, nitrogen-introducing tube, distillation head connected to a condenser, etc., was charged with 10.81 g (0.06 mole) of p-acetoxybenzoate, 3.88 g (0.02 mole) of 1,4-diacetoxybenzene, 2.16 g (0.01 mole) of 2,6-naphthalenedicarboxylic acid and 6.41 g (0.01 mole) of the diimidodicarboxylic acid obtained in Example 1, and nitrogen purging was carried out 3 times. Then, while the mixture was stirred moderately with flowing a small amount of nitrogen in the reactor, the temperature of the mixture was elevated to 200° C.

After the temperature reached 200° C, the stirring rate was increased, and the temperature of the mixture was increased stepwise to carry out reactions thereof at 240° C for 1 hour, 260° C for 1 hour, 280° C for 1 hour and 300° C for 1 hour. The amount of acetic acid distilled off during the reaction was 5.4 g.

Then, the pressure inside the reactor was decreased gradually, and while the pressure was maintained at a vacuume of 0.5 torr, the reaction mixture was further stirred at 320° C for 1 hour to complete polymerization.

Table 1 shows the physical properties of the resultant polymer.

REFERENTIAL EXAMPLES B and C, and COMPARATIVE EXAMPLES A and B

Polymers having a composition shown in Table 1 were synthesized by using the same reactor as that used in Referential Example A. Table 1 shows the physical properties of the polymers.

Comparative Examples A and B used conventionally known diimidodicarboxylic acids, and polymers obtained in these Comparative Examples showed low temperatures of 5 % weight decrease and had poor heat resistance.

TABLE 1

| | Referential Example | | | Comparative Example | |
|---|---|---|---|---|---|
| | A | B | C | A | B |
| Polymer Component (mole%) | | | | | |
| TPER | 10 | | | | |
| BAPP-DIDC | | 10 | | | |
| HFBAPP-DIDC | | | 10 | | |
| DDE-DIDC | | | | 10 | |
| HDA-DIDC | | | | | 10 |
| NDCA | 10 | 10 | 10 | 10 | 10 |
| HQ-DA | 20 | 20 | 20 | 20 | 20 |
| ABA | 60 | 60 | 60 | 60 | 60 |
| Tm (°C) | 291 | 287 | 288 | 267 | 270 |
| $T_{50/0}$loss | 438 | 441 | 438 | 427 | 415 |

Abbreviations for polymer components:

TPER : 4,4′-(1,3-diphenoxybenzene)-bis(N-trimellitimide)
BAPP-DIDC : {bis(4-Diphenyl ether)-2,2 propane}-bis(N-trimellitimide)
HFBAPP-DIDC : {bis(4-Diphenyl ether)-2,2-hexafluoro-propane}-bis(N-trimellitimide)
DDE-DIDC : 4,4′-Diphenyl ether-bis(N-trimellitimide)
HDA-DIDC : 1,6-Hexamethylene-bis(N-trimellitimide)
NDCA : 2,6-Naphthalenedicarboxylic acid
HQ-DA : 1,4-Diacetoxybenzene
ABA : p-Acetoxybenzoate

## Claims

1. A compound which is a diimidodicarboxylic acid represented by the formula

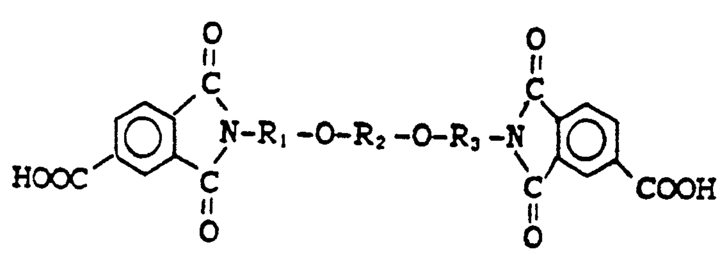

wherein $R_1$ and $R_3$ each represent an aromatic hydrocarbon having 6 carbon atoms, and $R_2$ represents an aromatic hydrocarbon having 6 carbon atoms, or an aromatic hydrocarbon having 12 carbon atoms which is optionally interrupted by a sulfonyl group, isopropylidene group or hexafluoroisopropylidene group.

2. A compound according to claim 1, represented by the formula

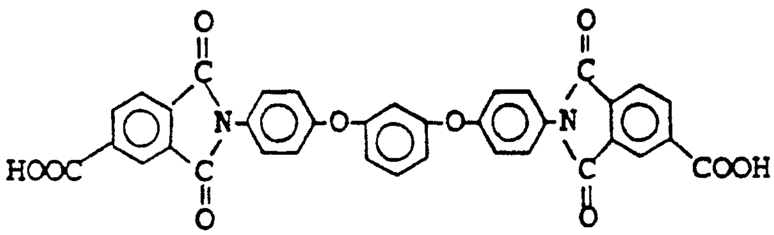

3. A compound according to claims 1, represented by the formula

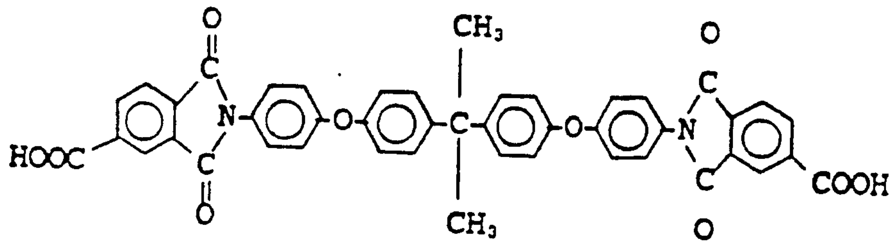

4. A compound according to claim 1, represented by the formula

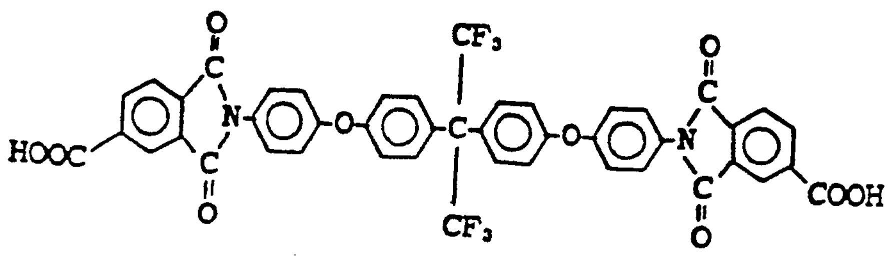

5. A compound according to claim 1, represented by the formula

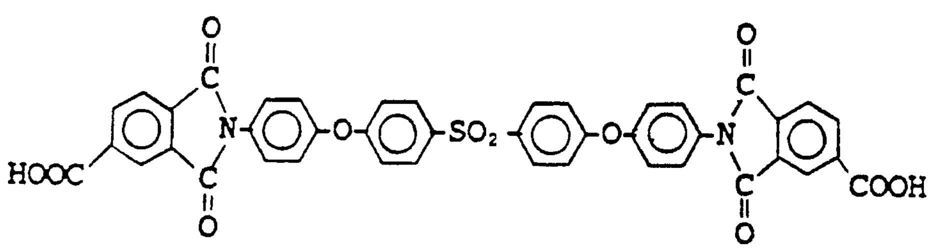

6. A compound according to claim 1, represented by the formula

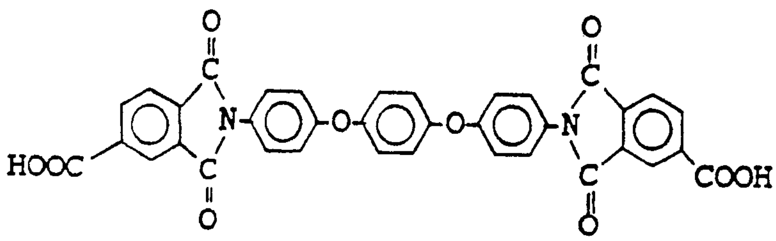

7. A compound according to claim 1, represented by the formula

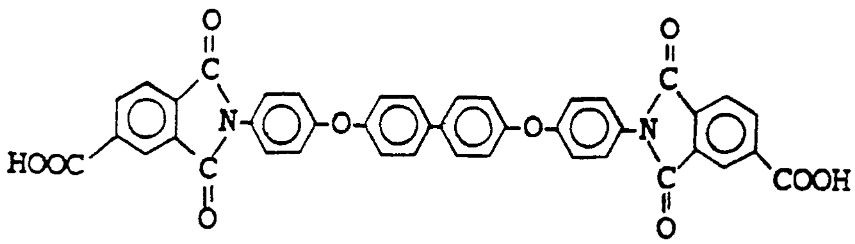

8. A process for the production of a compound as defined in claim 1, which comprises reacting a trimellitic anhydride with an aromatic diamine represented by the formula
$H_2N-R_1-O-R_2-O-R_3-NH_2$
wherein $R_1$ and $R_3$ each represent an aromatic hydrocarbon having 6 carbon atoms, and $R_2$ represents an aromatic hydrocarbon having 6 carbon atoms, or an aromatic hydrocarbon having 12 carbon atoms which is optionally interrupted by a sulfonyl group, isopropylidene group or hexafluoroisopropylidene group.

9. A process according to claim 8 which comprises dissolving the trimellitic anhydride in cresol and subsequently adding the aromatic diamine to the solution.

10. A polymer which is derived from a compound as defined in claim 1.

FIG. 1

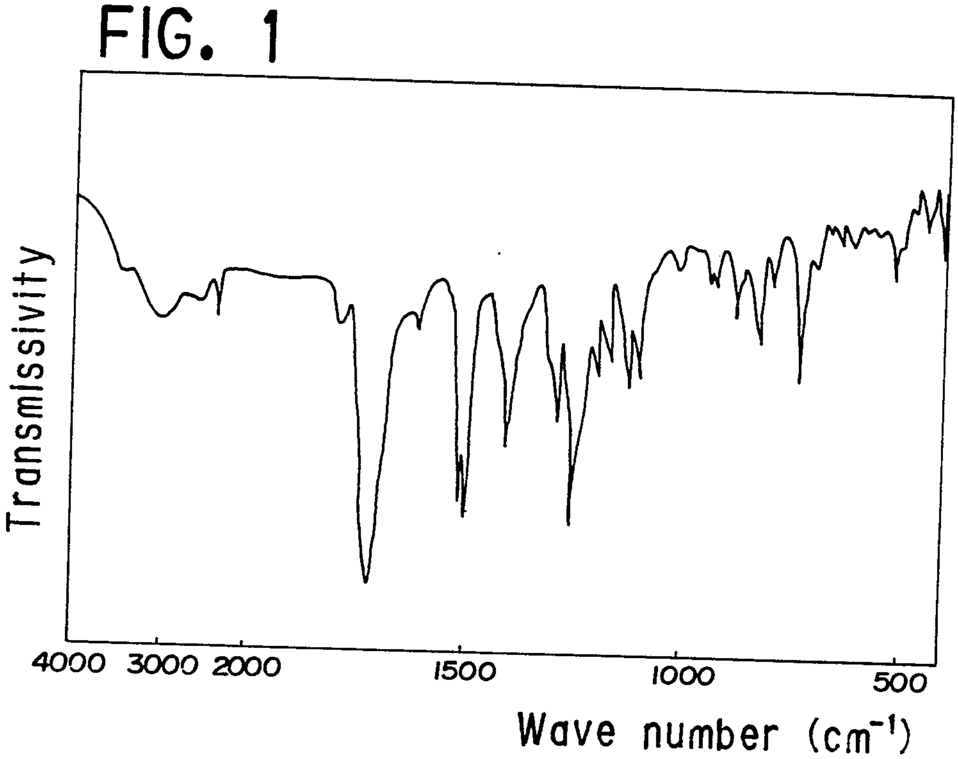


Infrared absorption spectrum

FIG. 2

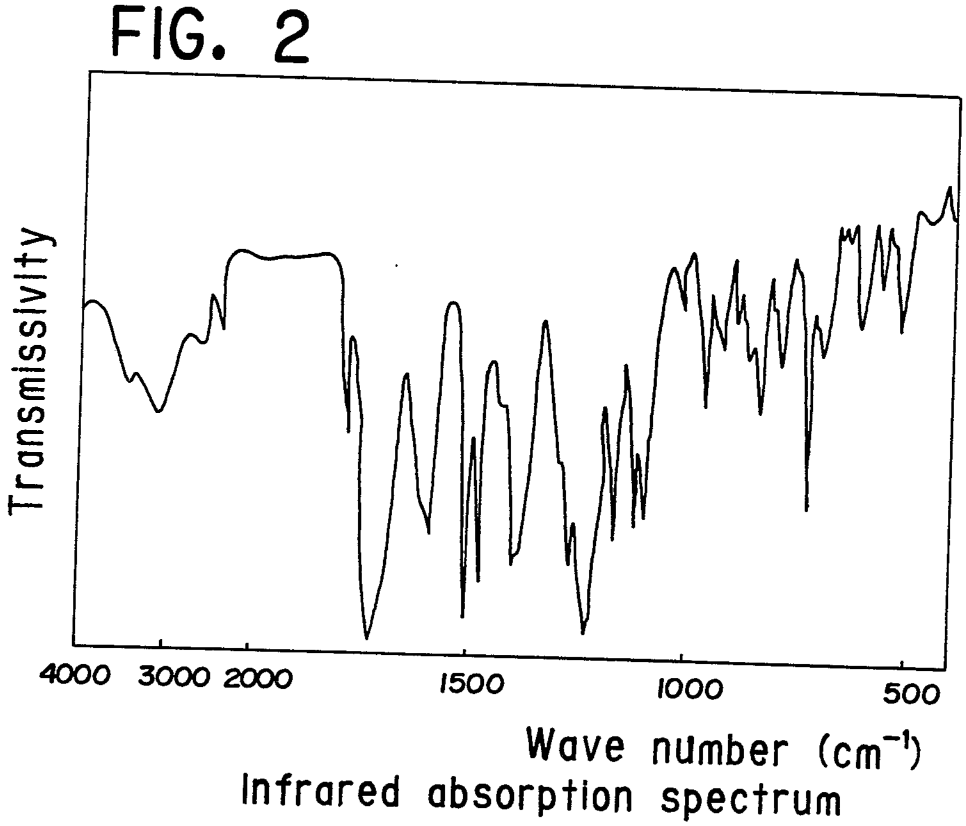


Infrared absorption spectrum

FIG. 3

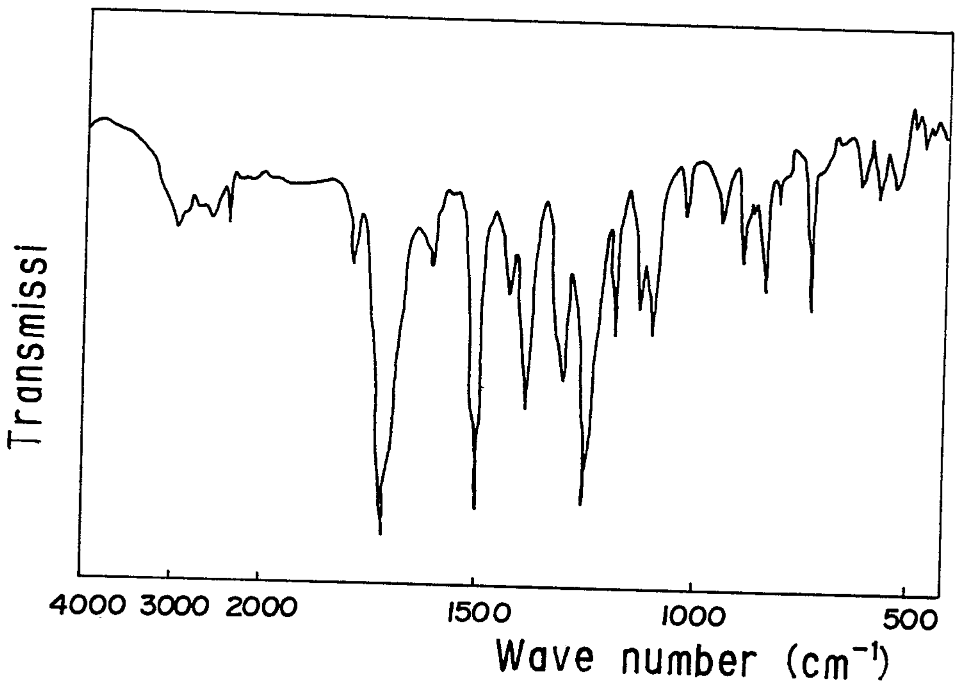


Infrared absorption spectrum

FIG. 4

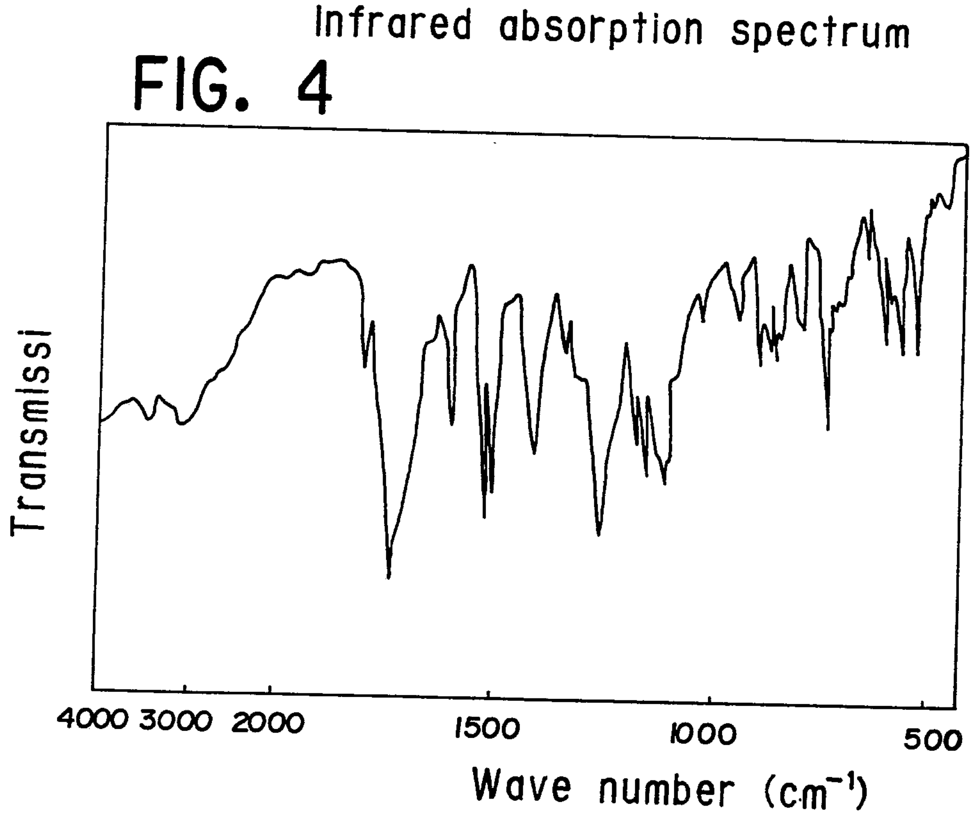


Infrared absorption spectrum

FIG. 5

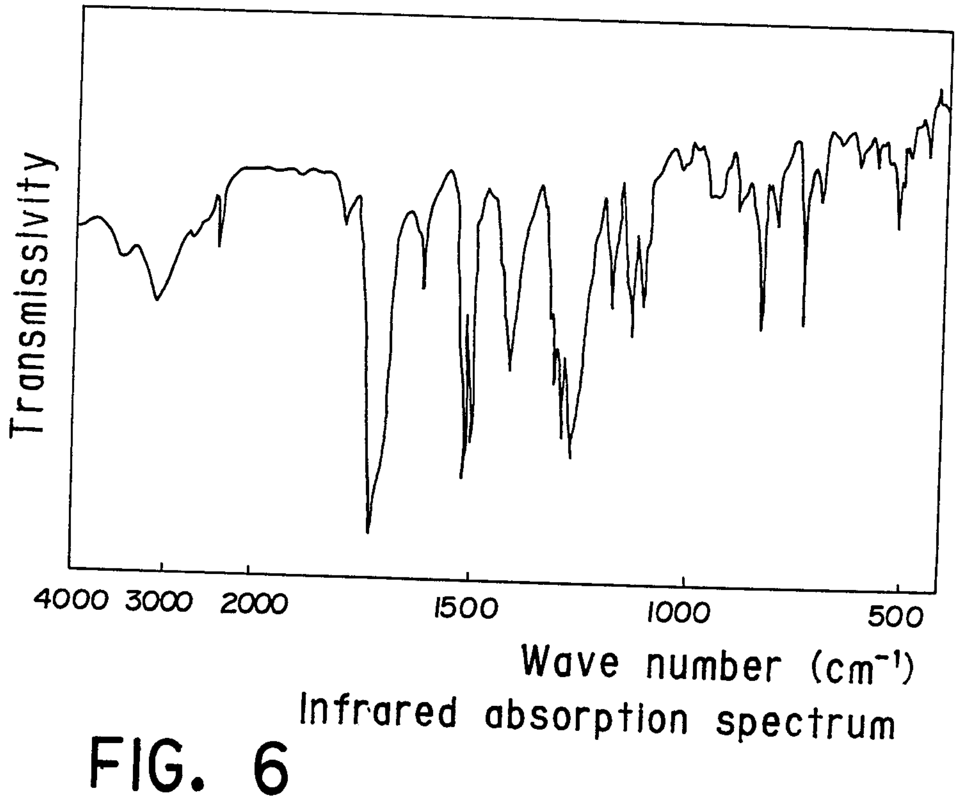
Transmissivity
4000 3000 2000 1500 1000 500
Wave number (cm⁻¹)
Infrared absorption spectrum

FIG. 6

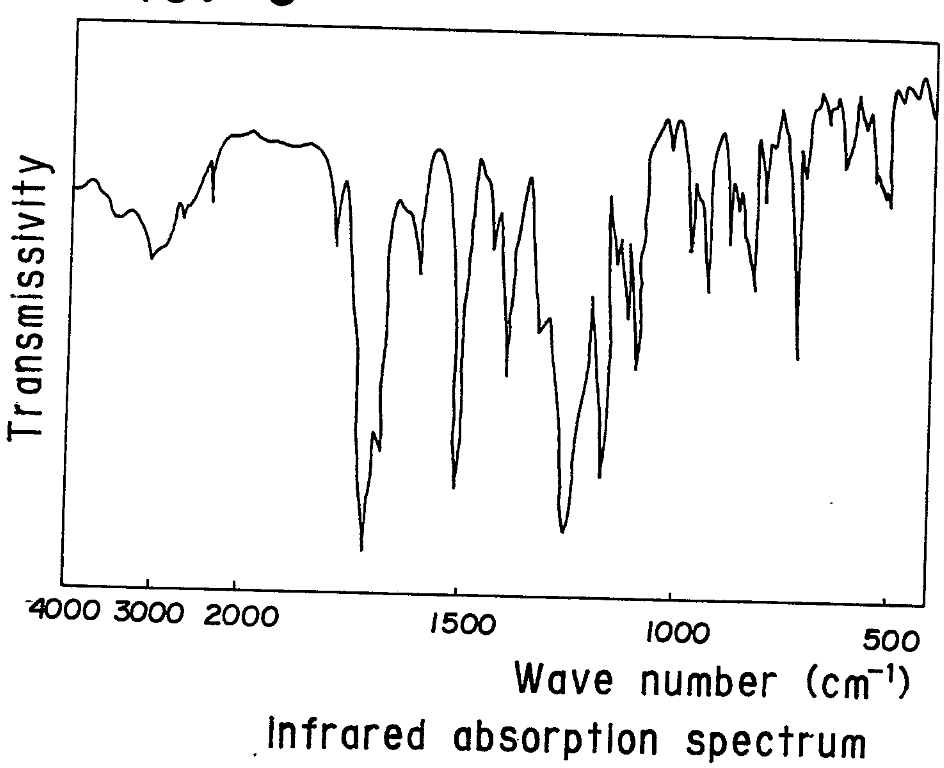
Transmissivity
4000 3000 2000 1500 1000 500
Wave number (cm⁻¹)
Infrared absorption spectrum

FIG. 7

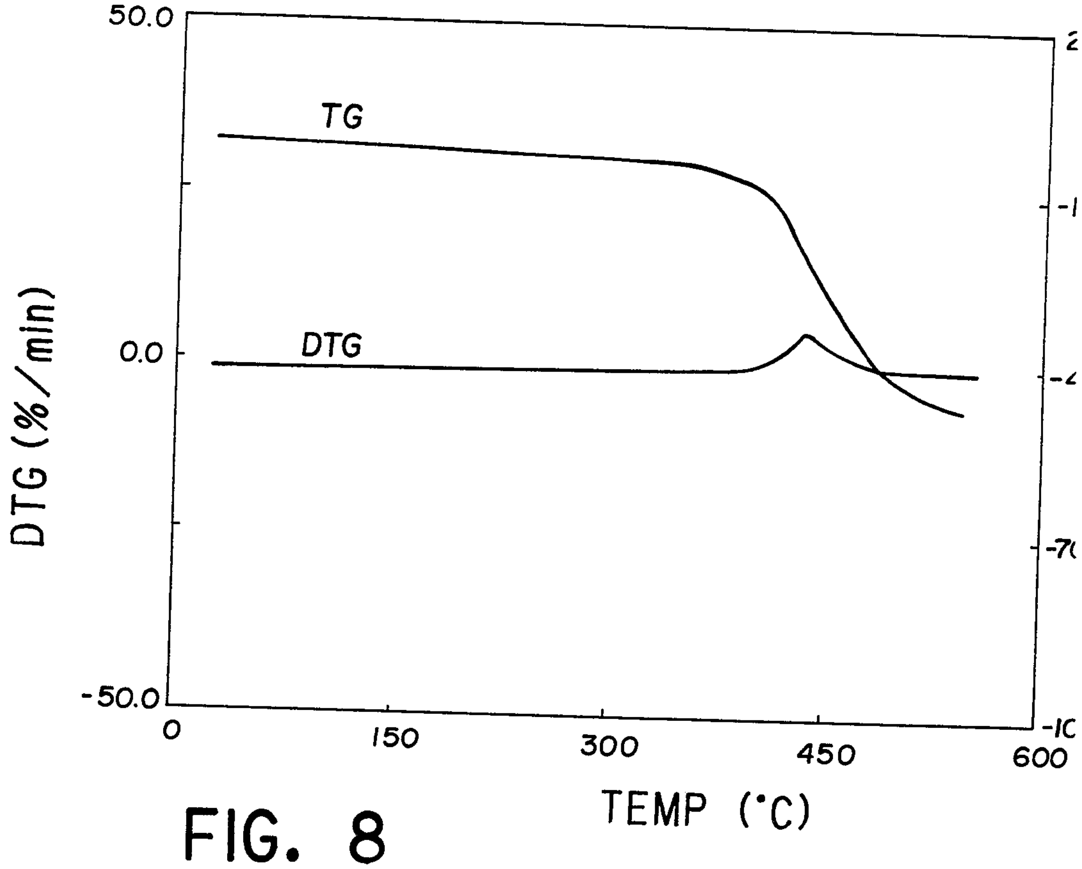
50.0
TG
DTG
0.0
-50.0
DTG (%/min)
0
150
300
450
600
TEMP (°C)

FIG. 8

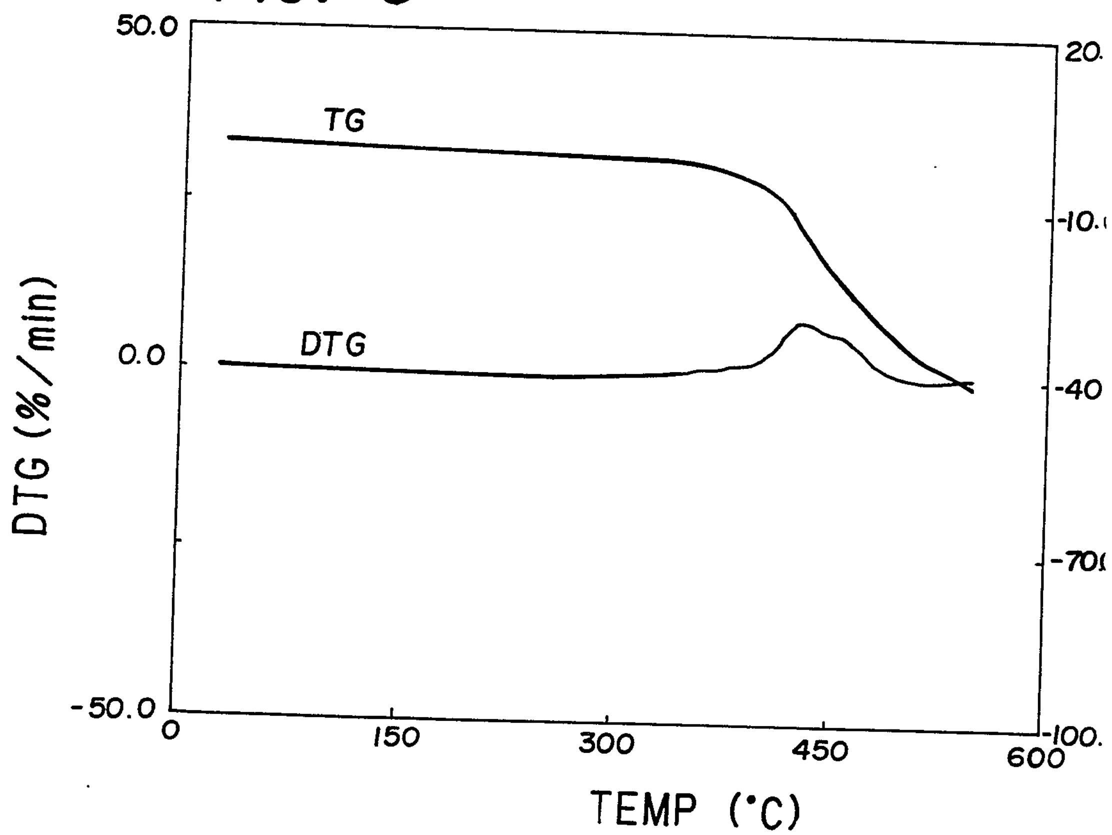
50.0
TG
DTG
0.0
-50.0
DTG (%/min)
20.
-10.
-40
-70
-100.
0
150
300
450
600
TEMP (°C)

FIG. 9

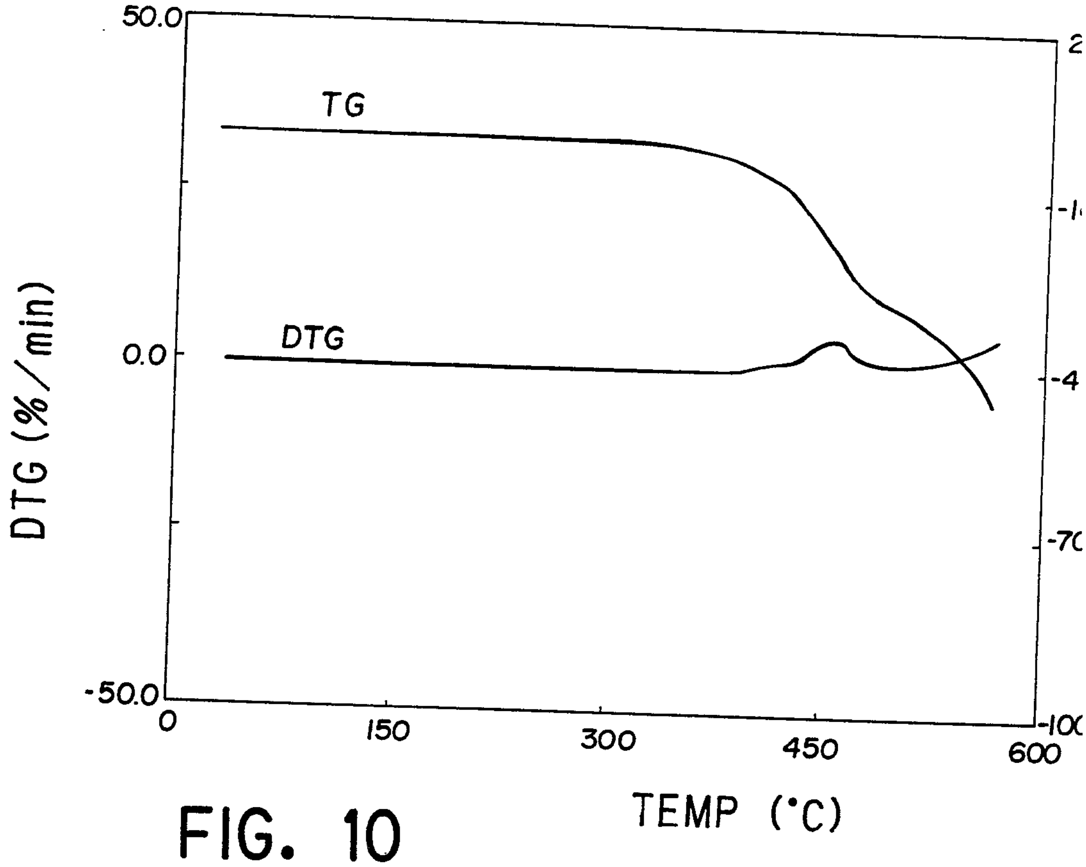
50.0
0.0
-50.0
DTG (%/min)
TG
DTG
0
150
300
450
600
TEMP (°C)

FIG. 10

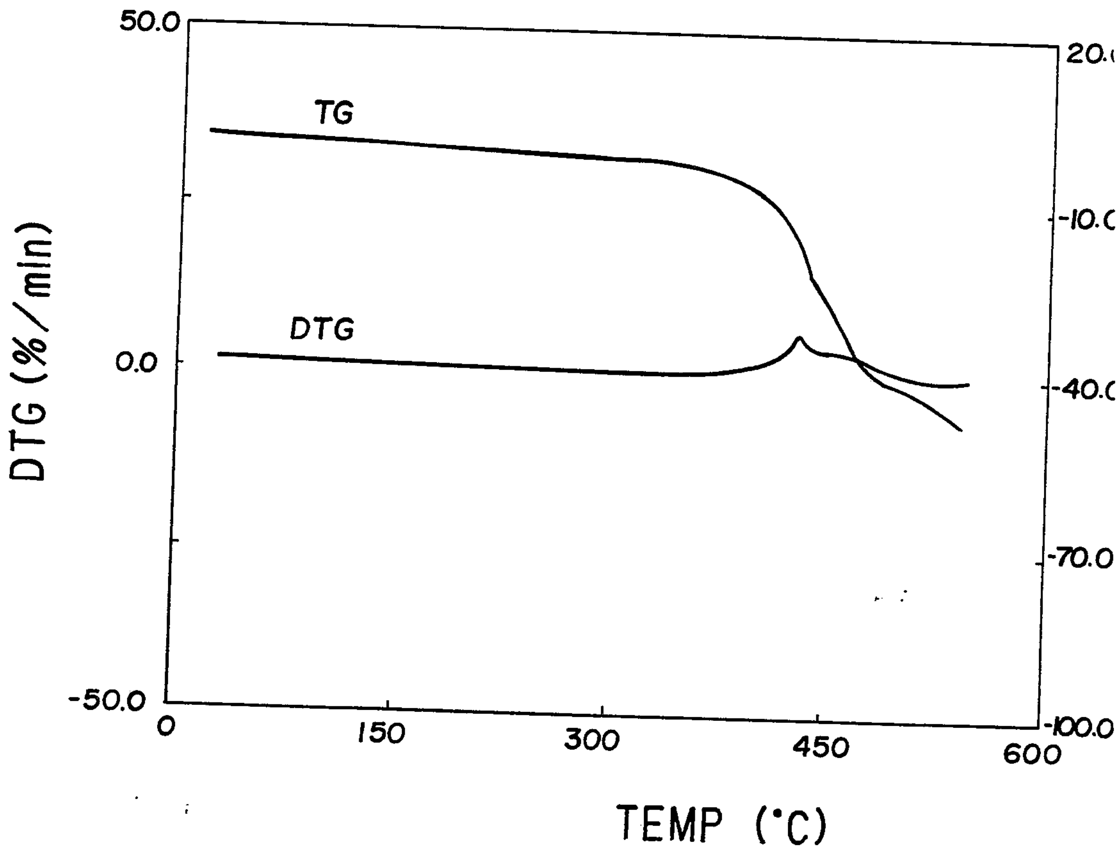
50.0
0.0
-50.0
DTG (%/min)
TG
DTG
20.0
-10.0
-40.0
-70.0
-100.0
0
150
300
450
600
TEMP (°C)

FIG. 11

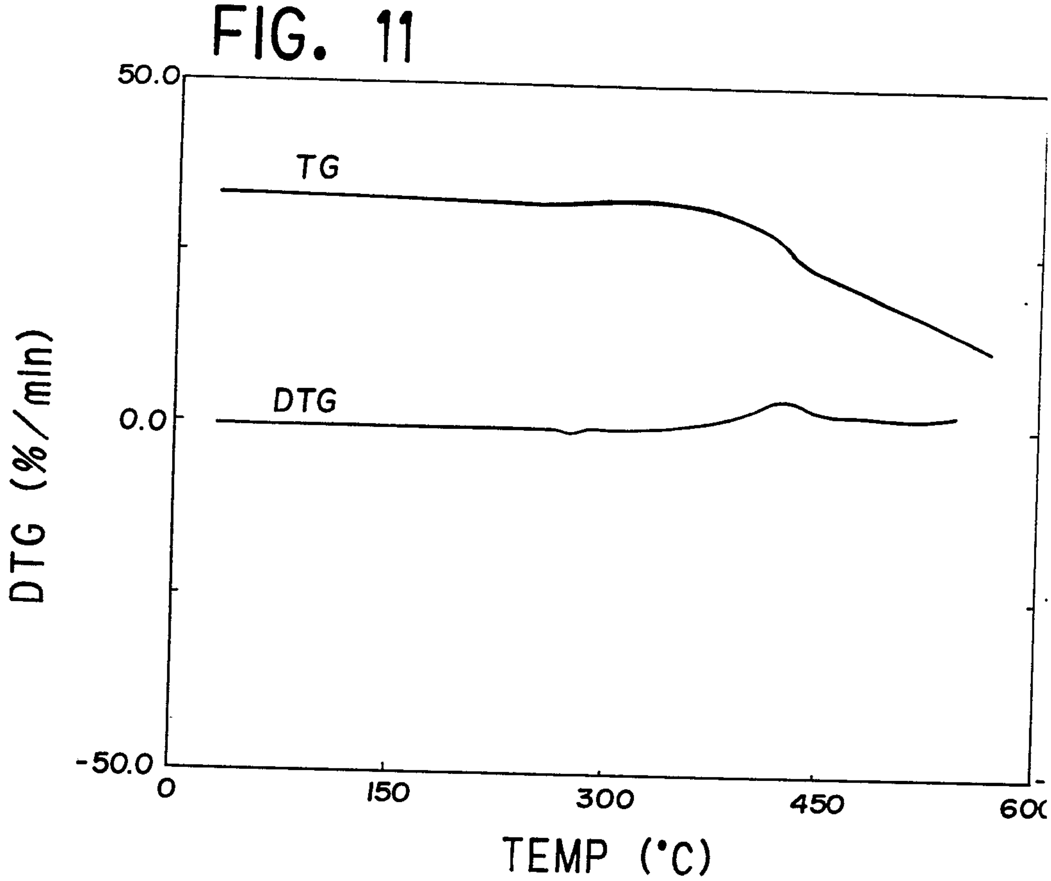
50.0
0.0
-50.0
DTG (%/min)
TG
DTG
0
150
300
450
600
TEMP (°C)

FIG. 12

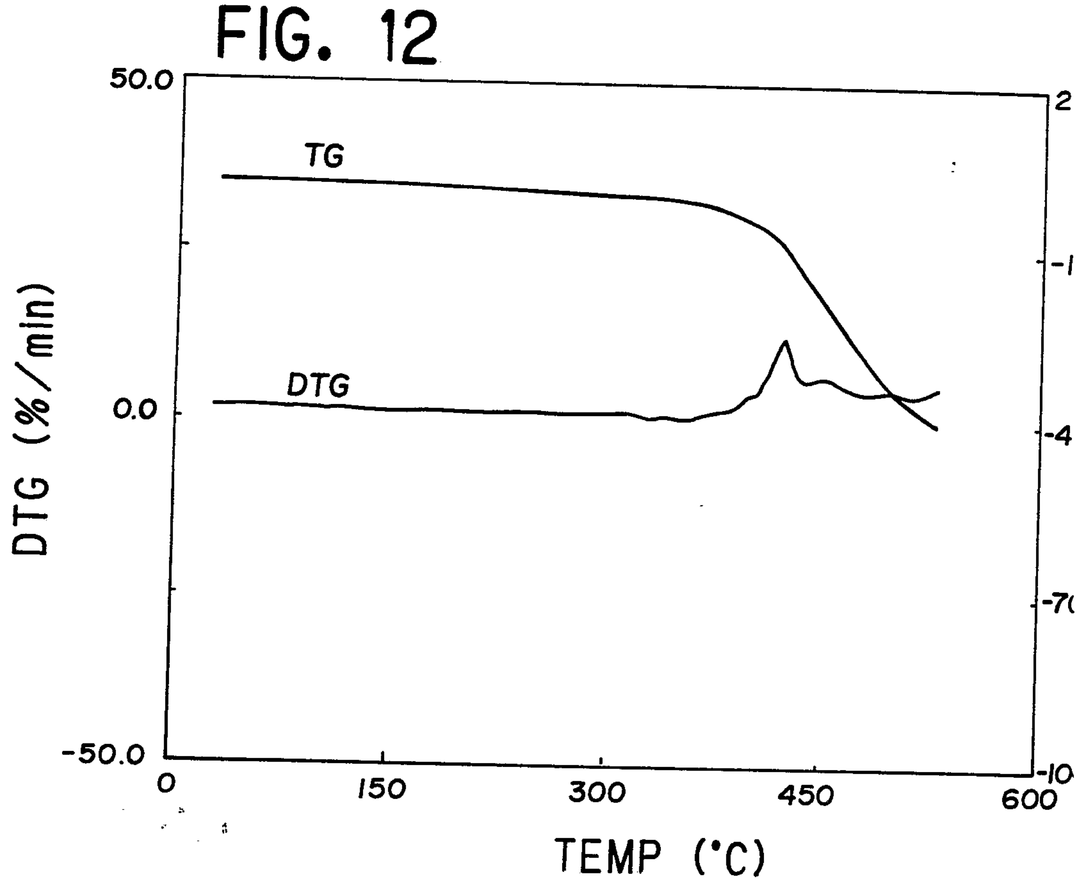
50.0
0.0
-50.0
DTG (%/min)
TG
DTG
0
150
300
450
600
TEMP (°C)

FIG. 13

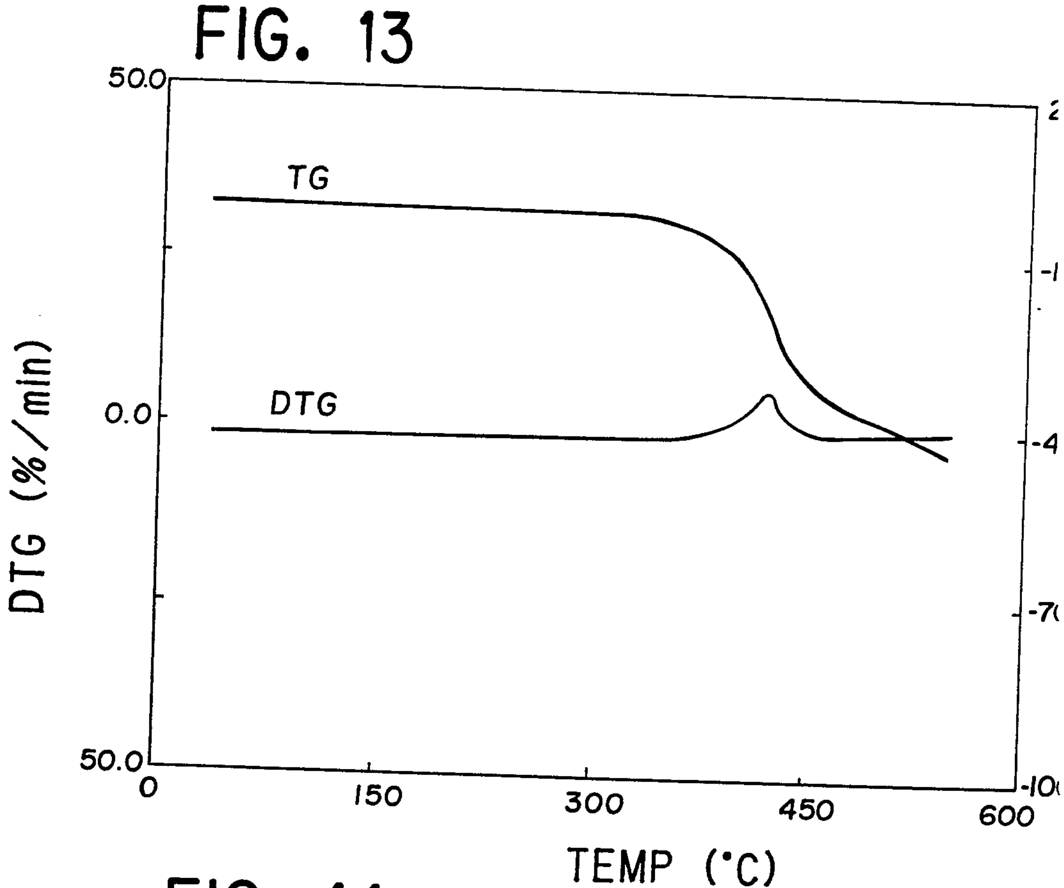
50.0
TG
DTG
0.0
DTG (%/min)
50.0
0
150
300
450
600
TEMP (°C)

FIG. 14

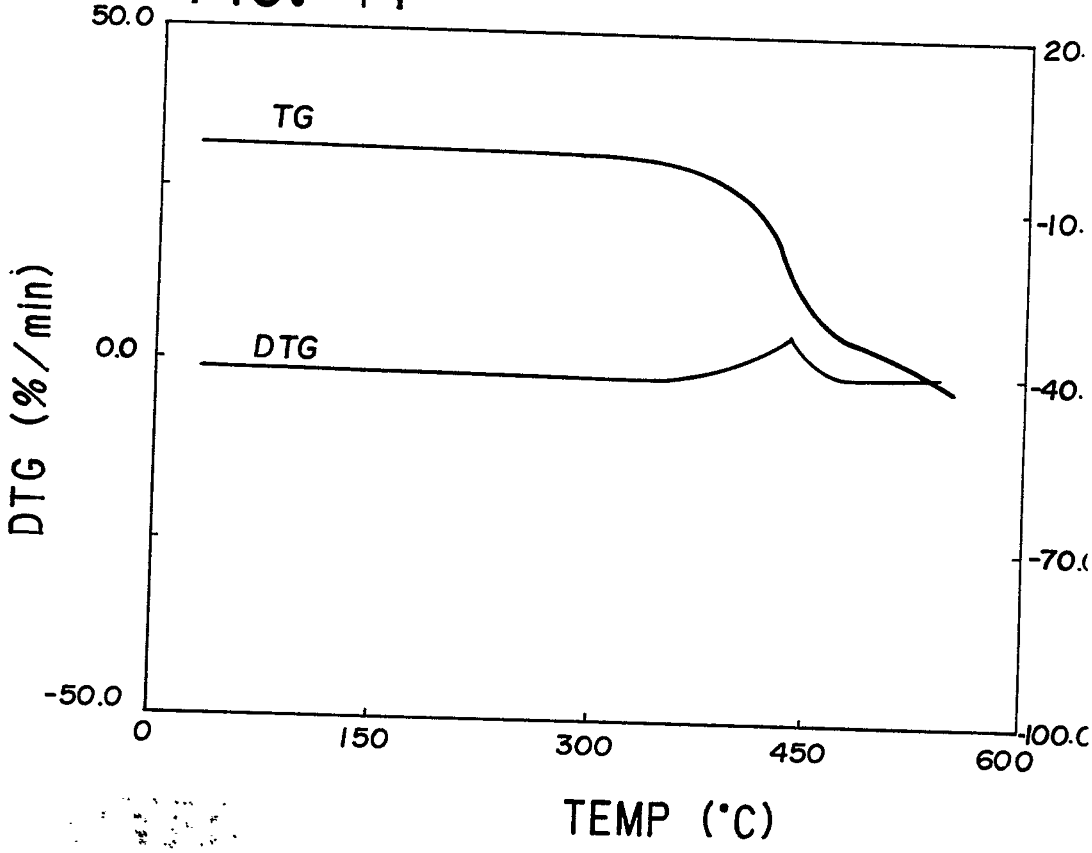
50.0
20.
TG
-10.
DTG (%/min)
0.0
DTG
-40.
-70.
-50.0
0
150
300
450
600
-100.
TEMP (°C)